# EUROPEAN PATENT APPLICATION

(11) **EP 3 569 698 A1**
(43) Date of publication of application: **20.11.2019**
(21) Application number: 18172615.9
(22) Date of filing: 16.05.2018
(51) Int. Cl.: C12N 5/077, A61K 35/32

(54) **PRODUCTS FOR THERAPY OF A MUSCULOSKELETAL CONDITION AND METHODS FOR THEIR PRODUCTION**

(71) Applicant: Veterinärmedizinische Universität Wien, 1210 Vienna (AT); Universität Wien, 1010 Vienna (AT); Universität für Bodenkultur Wien, 1180 Wien (AT)
(72) Inventor: JENNER, Florien, 1210 Vienna (AT); RIBITSCH, Iris, 1210 Vienna (AT); EGERBACHER, Monika, 1210 Vienna (AT); GERNER, Christopher, 1090 Vienna (AT); KREIL, David, 1190 Vienna (AT)
(74) Representative: Sonn & Partner Patentanwälte

(57) **Abstract**

The present invention provides a method for obtaining a fraction of a fetal cell culture supernatant, comprising the steps of obtaining a cell-containing sample of tissue (such as cartilage) or (cord-)blood or bone marrow from a non-human mammalian fetus, culturing the sample in a liquid cell culture medium, thereby obtaining a cell culture with a liquid supernatant, and isolating a fraction from the supernatant. Furthermore, a fraction obtainable by this method is provided. A pharmaceutical composition comprising this fraction is also provided, preferably for use in therapy, such as for use in a prevention or treatment of osteoarthritis, arthritis, tendinitis, tendinopathy, cartilage injury, tendon injury, rheumatoid arthritis, discospondylitis, meniscus injury, desmitis, desmopathy, intervertebral disc injuries, degenerative disease of intervertebral discs, reperfusion injury, wounds or inflammatory disease.

## Description

The present invention relates to products useful in the therapy of musculoskeletal conditions, such as osteoarthritis, and/or of reperfusion injury, wounds or inflammatory disease and methods for their production.

Osteoarthritis (OA), a degenerative joint disease characterized by progressive articular cartilage degeneration, is one of the most commonly diagnosed diseases in general practice and one of the leading causes of disability worldwide (Johnson and Hunter, 2014). In addition to its significant medical, social and psychological impact on quality of life, OA is associated with commensurate socioeconomic costs.

While OA has a multifactorial aetiopathogenesis involving genetic, molecular, and biomechanical influences as well as life-style and environmental stress stimuli, it culminates in a consistent molecular, structural and clinical sequence of disease progression, characterized by inflammation, gradual loss of proteoglycans, collagen type II (Col2) degradation, cartilage fibrillation, loss of maturational arrest and phenotypic stability of articular chondrocytes (as reviewed e.g. in Pap and Korb-Pap, 2015).

As adult articular cartilage has little intrinsic repair capacity and current treatment options are mostly palliative, the disease prevalence and burden place a strong emphasis on the need for new therapeutic strategies that could modify the structural progression of the disease and regenerate articular cartilage. The development of disease-modifying anti-OA drugs has thus far proven to be challenging due to the complexity of the disease and the pathophysiological pathways that drive disease progression. The same applies to other musculoskeletal conditions.

It is therefore an object of the present invention to provide new products suitable for the prevention or treatment of a musculoskeletal condition (such as OA) and/or of reperfusion injury, wounds or inflammatory disease and methods for their production or production of their precursor products.

The present invention provides a method for obtaining a fraction of a fetal cell culture supernatant. This method comprises the following steps:
- obtaining a cell-containing sample of tissue from a non-human mammalian fetus,
   - culturing the sample in a liquid cell culture medium, thereby obtaining a cell culture with a liquid supernatant, and
   - isolating a fraction from the supernatant (the fraction contains at least one bioactive factor such as a protein).

The invention further relates to a fraction which is obtainable by this method as well as a pharmaceutical composition comprising this fraction. This composition is suitable for therapy, in particular prevention or treatment of a musculoskeletal condition and/or of reperfusion injury, wounds or inflammatory disease.

The present invention further relates to cell supernatant fraction from non-human fetal cells, wherein the fraction comprises proteins, lipids, metabolites, extracellular vesicles and/or RNA, in particular miRNA. The present invention also relates to a pharmaceutical composition comprising this fraction. This composition is suitable for therapy, in particular prevention or treatment of a musculoskeletal condition and/or of reperfusion injury, wounds or inflammatory disease.

Cells secrete signalling molecules and other bioactive factors into their surroundings (especially in the context of proteomics, the entirety of secretions of a certain cell or cell type into the supernatant is called its "secretome"). When cells are cultured, these signalling molecules and other bioactive factors accumulate in the cell supernatant. In the course of the present invention it was found that the cell culture supernatant of cells obtained from fetal sheep cartilage, tendon, (cord-) blood or bone marrow differs markedly to that of the cell culture supernatant of cells obtained from adult sheep. In further experiments, this was confirmed independently for tendon. Fetal mammals, in contrast to adults, are much more capable of regenerating injured tissue such as cartilage or tendon. It is highly plausible that the cell supernatant of such fetal cell cultures or fractions thereof can be used therapeutically to increase the regenerative potential in adult tissues e.g. affected by musculoskeletal conditions and other degenerative conditions or injuries.

Fetal scarless regeneration is a paradigm for ideal tissue repair. Fetal mammals, in contrast to adults, are much more capable of regenerating injured tissues including skin, palate, tendon, bone and cartilage, especially in the first two trimesters of gestation (Al-Qattan et al., 1993; Degen and Gourdie, 2012; Kumahashi et al., 2004; Longaker et al., 1992; Longaker et al., 1990; Namba et al., 1998; Stone, 2000; Wagner et al., 2001; Walker et al., 2000). The mechanisms of this tightly regulated process, involving the interplay of growth factors, cytokines, proteinases, and cellular mediators combined with differences in cellular density, proliferation rate, inflammatory response, ECM composition and synthetic function compared to adults, are poorly understood in the art (Cowin et al., 1998a; Degen and Gourdie, 2012; Ferguson and O'Kane, 2004). In particular, the embryogenetic mechanisms of articular chondrogenesis remains largely unknown in the art (Decker et al., 2017; Jenner et al., 2014; Jenner et al., 2014; Lo et al., 2012) .

In the prior art, several attempts were made to create "stem-cell conditioned" media as well as pharmaceutical compositions prepared therefrom:
WO 2011/033260 A1 relates to stem-cell conditions medium compositions. According to an aspect of the document, there is provided a process for preparing a conditioned cell culture medium comprising: a) culturing eukaryotic cells in a growth medium having a composition effective to support cell growth; b) separating the cultured cells from the growth medium; c) maintaining the cultured cells in a basal medium having a composition suitable to maintain cell viability, but not to support substantial cell growth. Cells may be derived from adult, neonatal or foetal tissue and may be autologous or allogenic. However, it is neither clear whether these cells are actually obtained from a fetus (as being "derived" is vague and also refers to immortalised cell lines), e.g. by biopsy, nor whether these cells are non-human mammalian cells.

WO 2011/010966 A1 concerns pre-natal mesenchymal stem cells. A conditioned medium conditioned by such a pre-natal mesenchymal stem cell, cell culture or cell lines is described. The pre-natal mesenchymal stem cell, cell culture or cell line may comprise a cell line F1Ib, F2lb, F3lb, F1ki or F3li. These conditioned media are suggested to comprise cardioprotective activity and may be used to treat or prevent a range of cardiac disorders of diseases. The pre-natal cell from which the mesenchymal stem cell is derived may comprise a foetal cell. However, the term "derived" is vague and it is thus unclear whether the mesenchymal stem cell itself is actually obtained from a fetus.

The obtaining step of the inventive method may comprise an isolating or enrichment step. For instance, in the isolating step, the cells of the cell-containing sample may be separated from non-cell components (such as extracellular matrix components) of the cell-containing material. The non-cell-containing sample may be discarded whereas the cells may be used in the culturing step. Alternatively, or in addition thereto, in the enrichment step, certain cell types are enriched before the culturing step, e.g. by FACS.

Typically, the sample of tissue is obtained by in utero surgery or from aborted non-human foetuses.

Within the context of the present invention, the foetus is preferably a sheep foetus, a cow foetus, a horse foetus, a pig foetus, a goat foetus, a dog foetus, or a cat foetus. In embodiments, the foetus may be a rodent foetus such as a mouse foetus or rat foetus.

According to a particular preference, the foetus is within the first two trimesters of gestation, preferably within the first half of gestation. This increases regenerative potential of the fraction of the present invention.

According to a further preferred embodiment, the tissue or the cells is/are selected from cartilage, tendon, ligament, bone, bone marrow and (cord-)blood, which may be accessed by laparotomy followed by uterotomy of the mother animal. In particular, the tissue is articular cartilage. Such cartilage may be obtained for instance from a knee of the fetus.

In an embodiment of the inventive method, the culturing step is at least 1 hour, preferably at least 2 hours, especially at least 3 hours or even at least 4 hours (and may be extended to much longer times (weeks, months, years (e.g. for immortalized cells))). In addition, or alternatively thereto, the culturing preferably comprises less than 100 passages, preferably less than 50 passages, more preferably less than 20 passages, even more preferably less than 10 passages. Most typically, the culture is a primary culture. The culturing may be a 2D culture or a 3D culture. Typically, it is a 2D culture.

In the course of the present invention, it was found that the injured tissue response of fetal cells is different to the injured tissue response of adult cells. In addition, or alternatively thereto, the cells may be (e.g. chemically or mechanically) injured in culture. For instance, injury may be caused by compression, or pro-inflammatory factors such as TNF-alpha and IL1-beta may be added to the cells (see e.g. Sun et al., 2011, for details on a cartilage injury model).

According to a further preferred embodiment of the present invention, the tissue sample obtained comprises chondrocytes, chondroblasts, chondroprogenitor cells, tenocytes, tenoblasts, tendon progenitor cells, fibrocytes, fibroblasts, fibrochondrocytes, fibrochondroblasts, synoviocytes, synovioblasts, osteocytes, osteoblasts, osteoclasts, hepatocytes, monocytes, macrophages, mesenchymal stem cells, mesenchymal progenitor cells and/or interzone cells.

In a further preferred embodiment of the present invention, the isolated fraction comprises proteins, lipids, metabolites, extracellular vesicles, and/or RNA, in particular miRNA. Most typically, these (e.g. the proteins and/or the extracellular vesicles) are secreted from the cells of the cell culture into the supernatant.

According to a particular preference, the fraction is a whole-protein fraction of the supernatant or a total solids fraction of the supernatant, as obtained e.g. by lyophilisation.

Any cell culture medium suitable for mammalian cell culture can be used for the present invention. Preferably, the liquid cell culture medium used in the inventive method is a serum-free cell culture medium, a protein free cell culture medium or a chemically defined cell culture medium. The cell culture medium may be a medium with or without FCS or other nutrient additives. Alternatively, or in addition thereto, the cell culture medium preferably does not comprise any factor (e.g. such as the ones mentioned above) which are also secreted by the cells of the cell culture (i.e. before use in the inventive method).

According to a preferred embodiment, the isolating step of the inventive method comprises a sterile filtration. The isolating step may further (or alternatively) comprise the addition of a protein-precipitating agent, such as ethanol or ammonium sulphate.

According to a further preferred embodiment, the isolating step comprises a centrifugation step. For instance, the isolating step may comprise two centrifugation steps: first centrifugation step to separate the supernatant from cell debris or cells that have become unattached from the cell culture vessel, and a second centrifugation step performed after precipitation of the soluble factors such as proteins in the supernatant to separate the solid secreted factors from the solvent.

According to a particular preference, the isolating step comprises a preservation step, such as the addition of a stabilising or antioxidant agent. The preservation step may also be a drying step, especially lyophilisation.

According to particular preferred embodiment, this fraction is dry, preferably lyophilised.

In a further aspect, the present invention relates to a pharmaceutical composition, comprising or consisting of the fraction described hereinabove.

In the context of the present invention, the expression "pharmaceutical composition" refers to any composition comprising at least one active agent (e.g. the fraction of the present invention), and preferably one or more excipients, which is pharmaceutically acceptable for administration (in particular for topical administration or intravenous administration) to an individual, especially a mammal, in particular a horse or a human. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives, such as benzalkonium chloride. The pharmaceutical composition according to the present invention may be liquid or ready to be dissolved in liquid such as sterile, deionised or distilled water, or sterile isotonic phosphate-buffered saline (PBS). Preferably, 1000 µg (dry-weight) of such a composition comprises or consists of 0.1-990 µg, preferably 1-900µg, more preferably 10-200µg dried fraction of the present invention, and optionally 1-500 µg, preferably 1-100 µg, more preferably 5-15 µg (buffer) salts (preferably to yield an isotonic buffer in the final volume), and optionally 0.1-999.9 µg, preferably 100-999.9 µg, more preferably 200-999 µg other excipients. Preferably, 100 mg of such a dry composition is dissolved in sterile, deionised/distilled water or sterile isotonic phosphate-buffered saline (PBS) to yield a final volume of 0.1-100 ml, preferably 0.5-20 ml, more preferably 1-10 ml. The dosage and method of administration, however, typically depends on the individual to be treated. In general, the dried fraction (within the composition) may be administered at a dose of between 1 µg/kg body weight and 100 mg/kg body weight, more preferably between 10 µg/kg and 5 mg/kg, most preferably between 0.1 mg/kg and 2 mg/kg. The pharmaceutical composition may for instance be provided as injectable solution, topical solution, liquid, tincture, gel, ointment, balsam, cream, powder, liniment, lotion, patch or matrix for local or parenteral administration, e.g. to treat injuries, degenerative and/or inflammatory conditions. In embodiments, the pharmaceutical composition may be injected into a joint or close to/into the tendon of the patient (mammal, in particular human or horse), e.g. into the joint or close to/into the tendon afflicted by one of the musculoskeletal conditions described herein.

In particular, the pharmaceutical composition of the present invention is for use in a prevention or treatment of osteoarthritis, arthritis, tendinitis, tendinopathy, cartilage injury, tendon injury, rheumatoid arthritis, discospondylitis, meniscus injury, desmitis, desmopathy, intervertebral disc injuries, degenerative disease of intervertebral discs, reperfusion injury, wounds or inflammatory disease. For instance, the pharmaceutical composition may be administered to a mammal, preferably a sheep, a cow, a horse, a pig, a coat, a dog, a cat, or a human, in need thereof, such as mammal having or at risk of developing or predisposed of developing any one of the conditions or diseases mentioned above.

As used herein, "prevention" should not be interpreted as an absolute success in the sense that a patient can never develop an associated disease, reaction or condition but as the reduction of the chance of developing the disease, reaction or condition in a prophylactic treatment. Prevention by prophylactic treatment is to be understood in the sense of a reduction of the risk of development not as a total risk avoidance.

As used herein, the term "tissue" also includes blood (e.g. cord blood) and bone marrow.

The present invention is further illustrated by the following figures and examples, without being limited thereto.

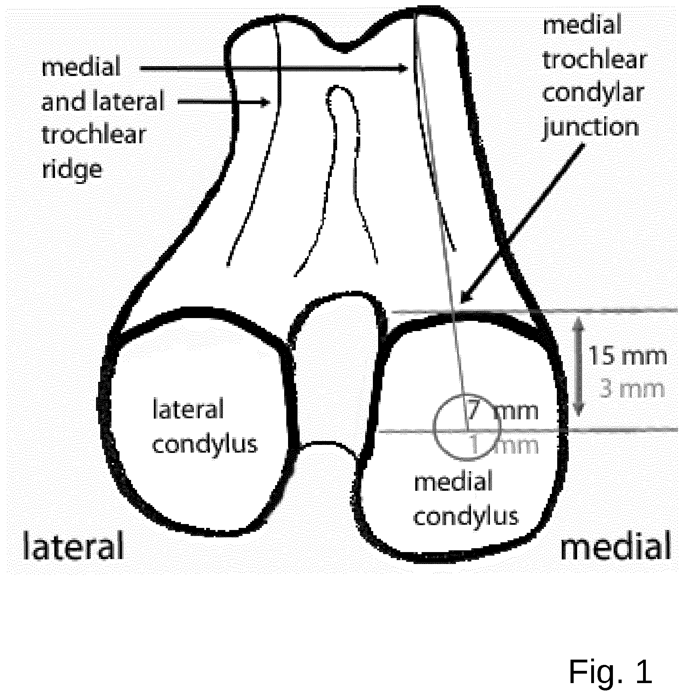

**Figure 1****:** Diagram of the distal femur with the medial and lateral trochlea ridge and the medial and lateral condyle identified as landmarks. Cartilage lesion location and size is indicated in blue in in adult and in green in fetal sheep. The lesion was centred 15 mm (adult) resp. 3 mm (fetus) distant from the medial trochlear-condylar junction on a line, which virtually extended the medial trochlear ridge.

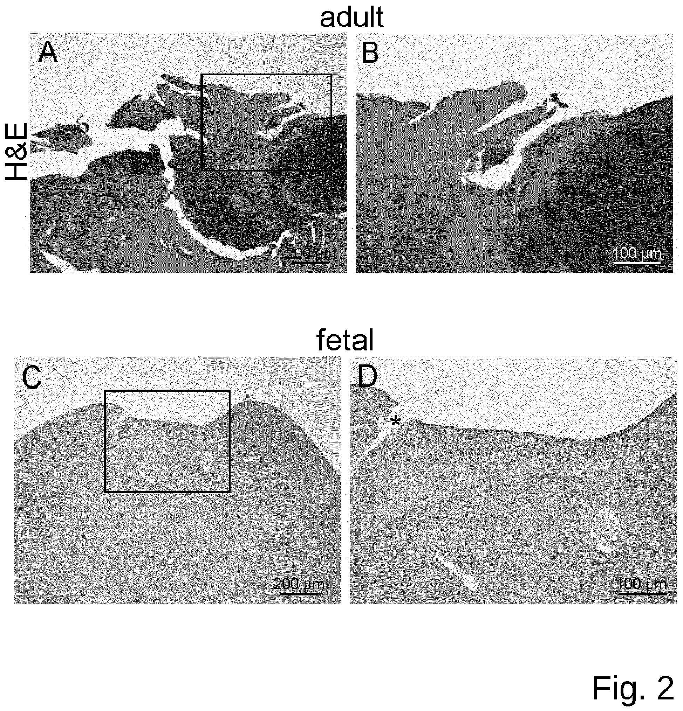

**Figure 2****:** Healing of adult (5 months post injury) and fetal (28 days post injury) cartilage defects: Haematoxylin and Eosin (H&E) stained sections. Adult (A and B): no repair except in areas of micro fracture, tissue mixture of fibrocartilage with partial hyalinisation, no integration with surrounding cartilage (see insert). Fetal (C and D): defect filled to 80 - 90% with differentiating hyaline cartilage and the superficial 10-20% with repair tissue with progressing hyalinisation, good integration with surrounding cartilage, processing artefact (*).

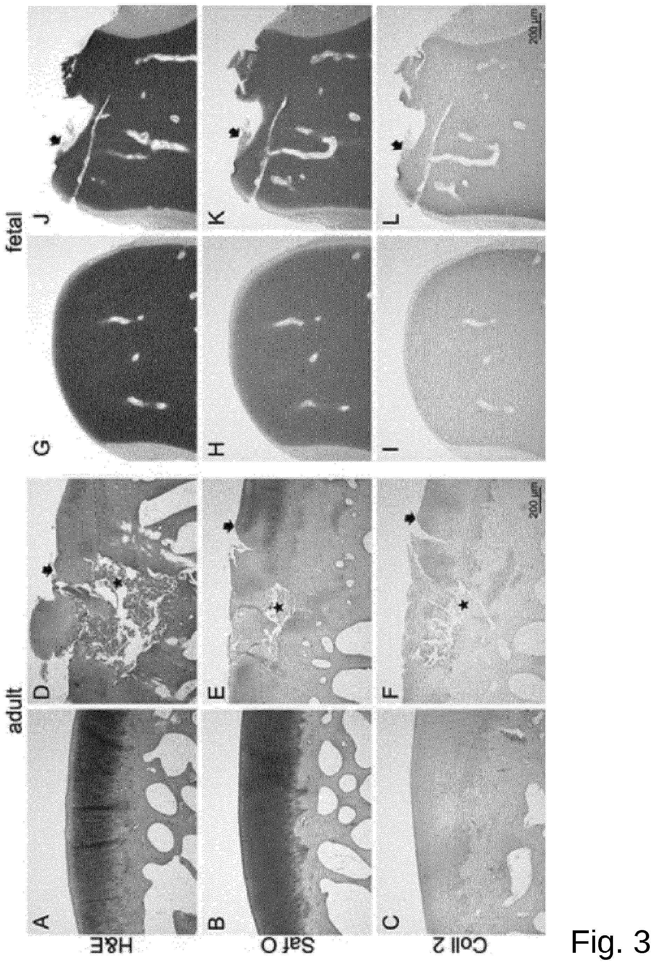

**Figure 3****:** Morphology and extracellular matrix composition of healthy and injured adult and fetal cartilage: Haematoxylin and Eosin (H&E), Safranin O, and collagen type 2 (Col2) staining. Arrows mark the transition from healthy cartilage to the lesion site; asterisks indicate sites of microfracture penetrating the subchondral bone plate in D, E, F. Fibrous tissue partly covering the surface of the lesion (arrows in J, K, L) was found in fetal injured condyles.

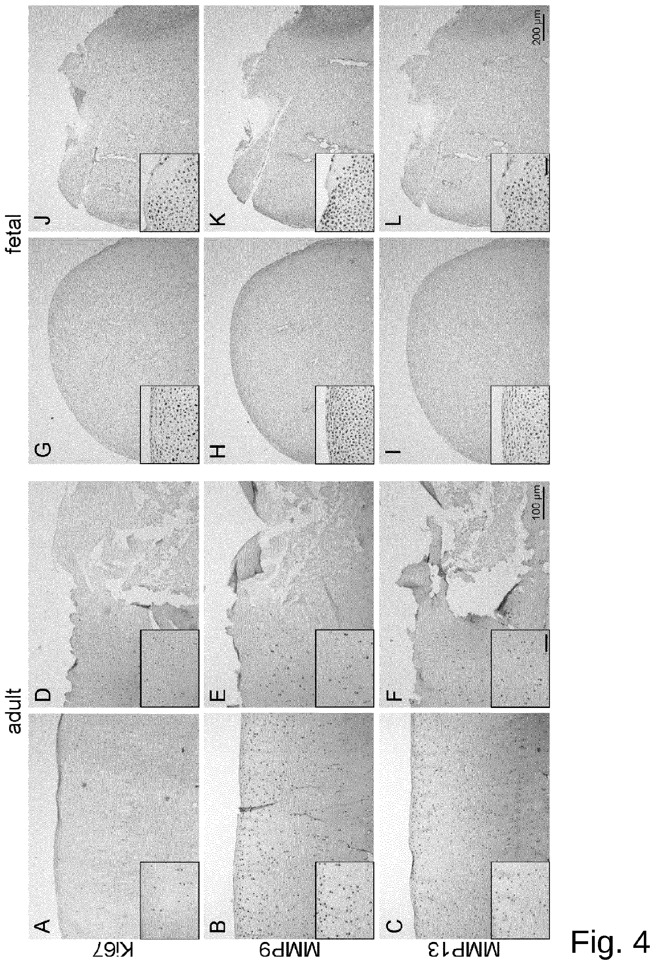

**Figure 4****:** Distribution of proliferation marker Ki67 (A, D, G, J) and matrix metalloproteinases (MMPs, B, C, E, F, H, I, K, L) in healthy and injured adult and fetal cartilage. Inserts in fetal injured condyles indicate transition from healthy cartilage to the lesion site. Scale bar in adult samples = 100 µm, scale bar in fetal samples = 200 µm and scale bar in inserts = 20 µm.

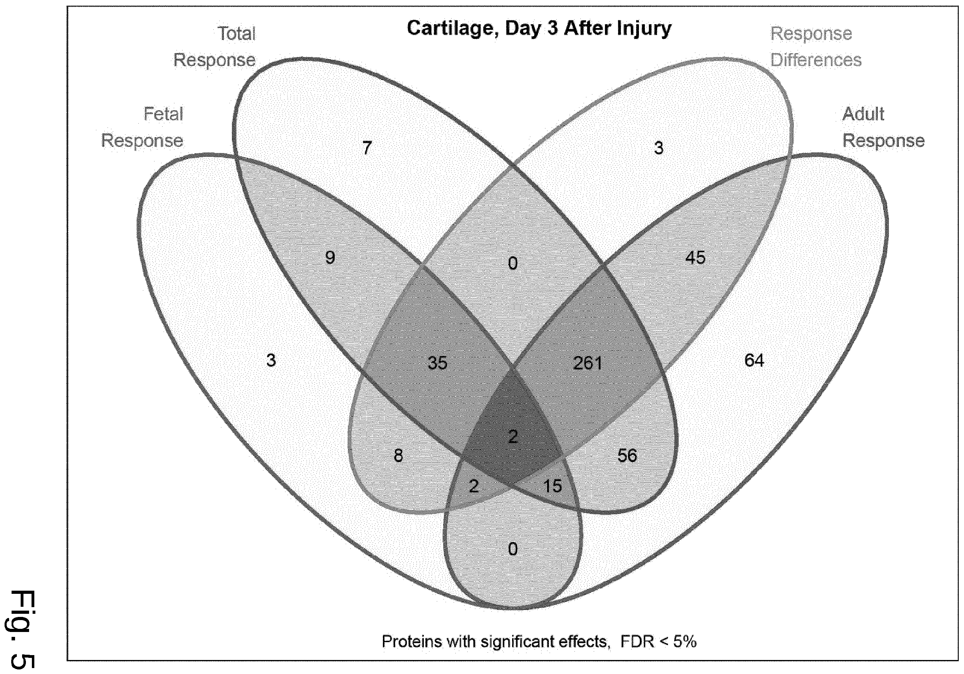

**Figure 5****:** The Venn diagram gives an overview of genes implicated by a range of differential screening tests (n= 3 biological replicates/group, 2 technical replicates/biological replicate). Specifically, we examine the average Total Response to injury (magenta), the Fetal Response (blue), the Adult Response (red), and significant Response Differences (green). Separately assessing significances for each of the four tests improves sensitivity and specificity by avoiding an accumulation of thresholding artefacts. Comparing cartilage on day 3 after injury with matching control tissues yielded 385 genes implicated in the total response incorporating the average evidence from all sample types (7+9+0+35+261+2+56+15). Analogously, 74 genes were implicated in the fetal response (9+3+35+8+2+2+15), of which 13 were newly identified (3+8+2+0). Conversely, 445 genes were implicated in the adult response (45+261+64+2+56+2+15), of which 111 were newly identified (45+64+2+0). Response differences are shown by 356 genes with an injury response in fetal samples that was significantly different to that in adult samples (3+0+45+35+261+2+8+2), including 3 previously not implicated genes, 8 genes so far only implicated in the fetal response, 45 genes so far only implicated in the adult response, 2 genes already implicated in both, 35 genes already implicated in the total and the fetal responses, 261 genes already implicated in the total and the adult responses, and 2 genes implicated in all, reflecting that response strength and direction of expression change can be affected differently in the injury response of fetal and adult samples.

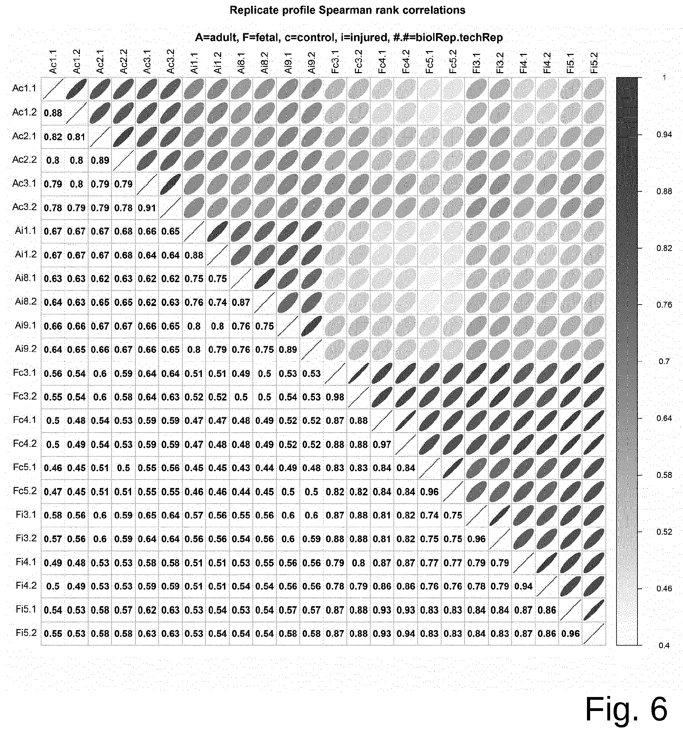

**Figure 6****:** Sample correlation structure. This figure compares pairwise sample correlations, with Spearman rank correlation coefficients given in the boxes below the diagonal, which are visualized above the diagonal, with darker and narrower ellipses indicating higher correlations. Rows and columns show sample labels, where A/F=adult/fetal, c/i=control/injured, and #.# show biological and technical replicate numbers (n= 3 biological replicates/group, 2 technical replicates/biological replicate).

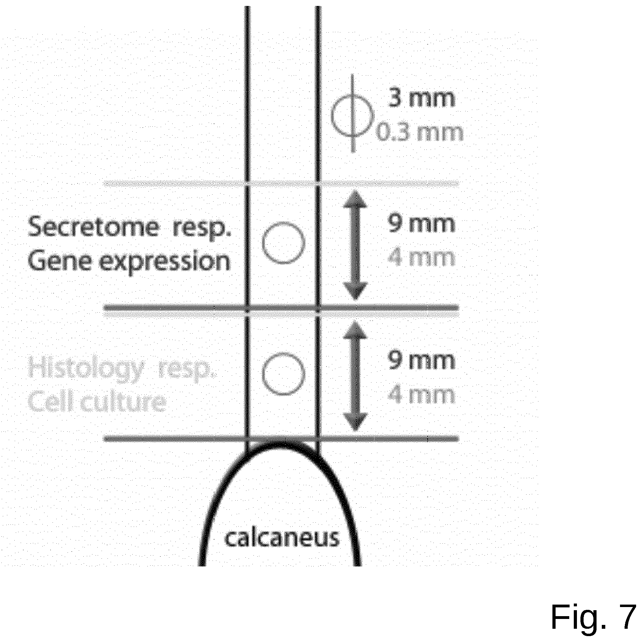

**Figure 7****:** Diagram of the superficial digital flexor tendon of the gastrocnemius tendon bundle with the calcaneus identified as landmark. Tendon lesion location and size is indicated in blue in in adult and in green in fetal sheep. The distal lesion was placed 9 mm (adult) resp. 4 mm (fetus) proximal to the calcaneus, the proximal lesion at a distance of 9 mm (adult) resp. 4 mm (fetus) proximal to the distal lesion.

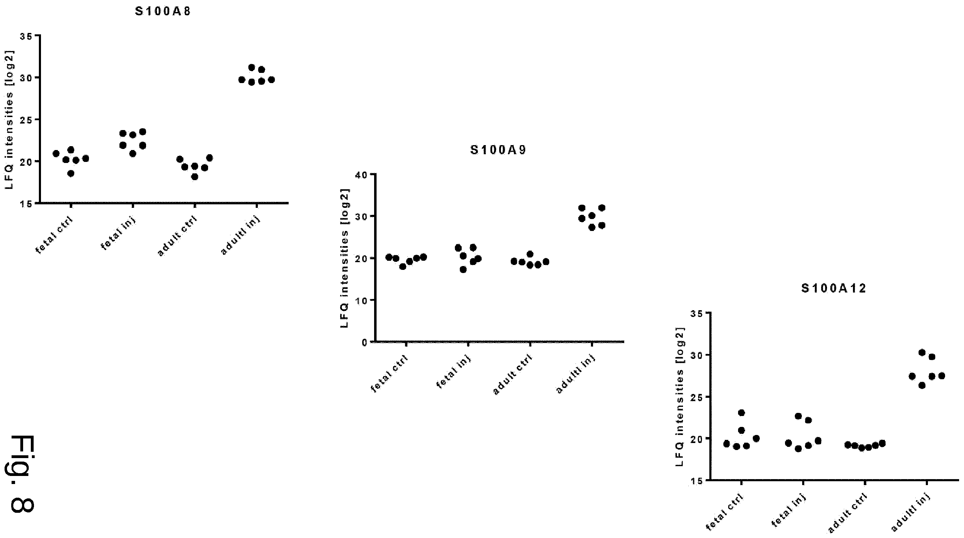

**Figure 8****:** Proinflammatory factors S100A8, S100A9 and S100A12 were clearly upregulated in the supernatant of adult injured tendon whereas they remained essentially unchanged in the supernatant of fetal tendon upon injury (ctrl: control, inj: injured).

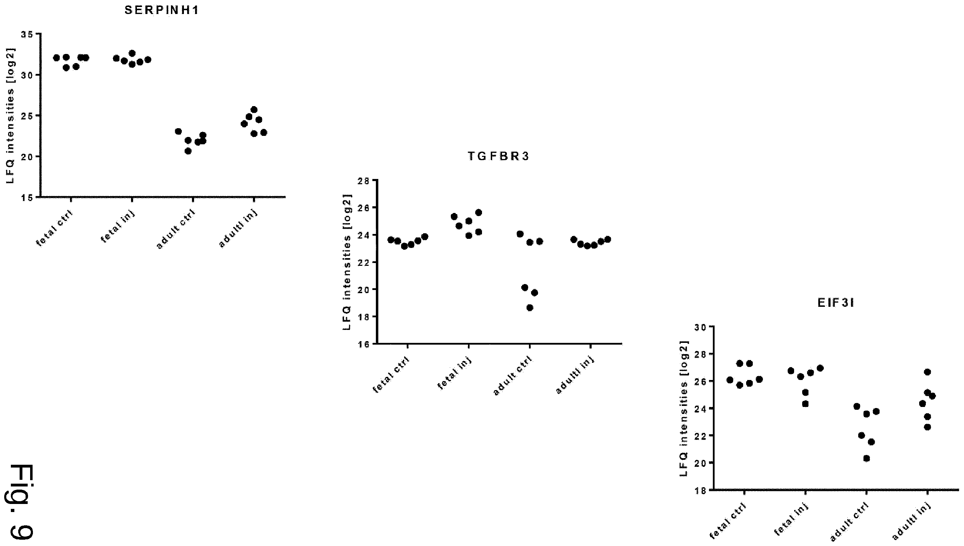

**Figure 9****:** Of the growth factors SERPINH1, TGFBR3 and EIF3I, the factor SERPIN1 was clearly upregulated in the supernatant of fetal tendon. (ctrl: control, inj: injured).

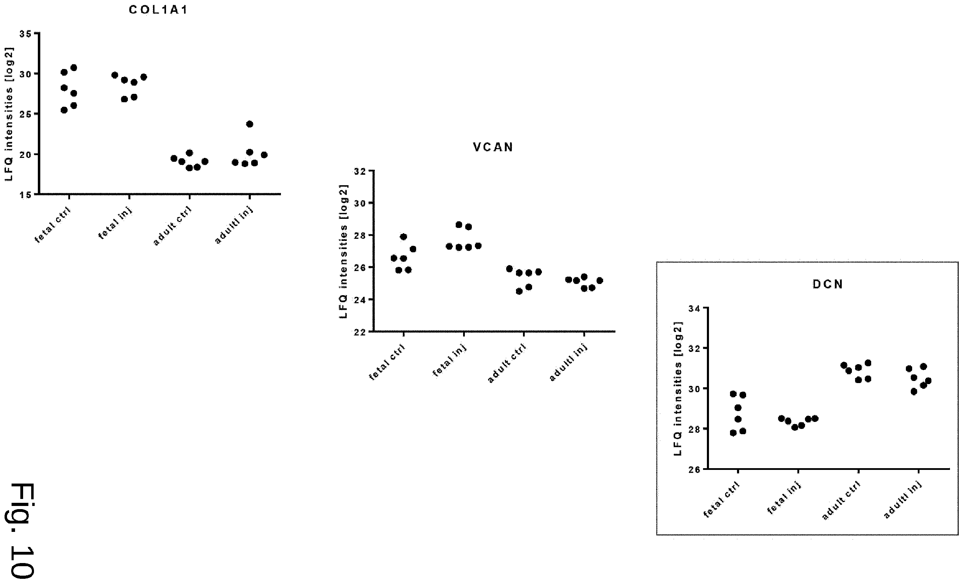

**Figure 10****:** Of the extracellular matrix components collagen 1 A1 (Col1A1, versican (VCAN) and decorin (DCN), Col1A1 was clearly upregulated in the supernatant of fetal tendon, VCAN was upregulated in the supernatant of fetal tendon and even increased upon injury, whereas DCN was lower in the supernatant of fetal tendon. (ctrl: control, inj: injured).

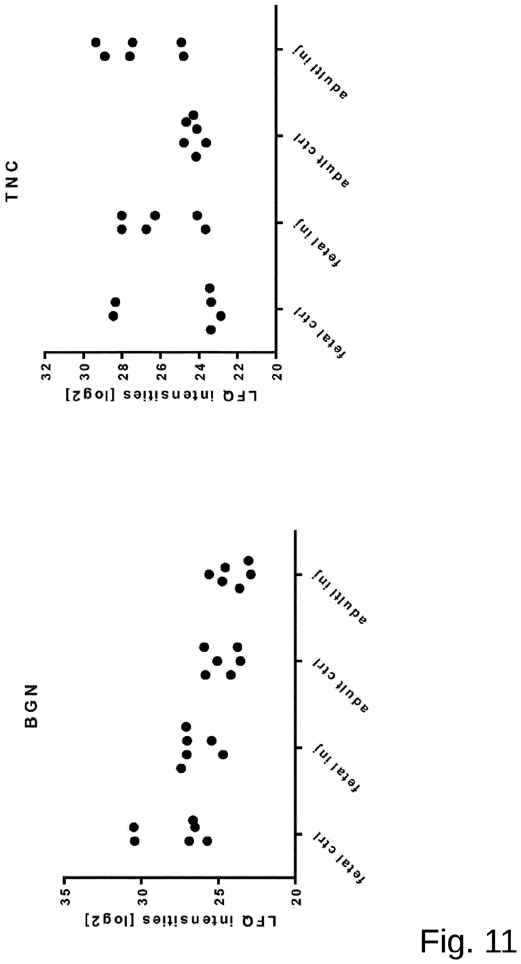

**Figure 11****:** The extracellular matrix components biglycan (BGN) and tenascin-C (TNC) exhibited a mixed pattern. (ctrl: control, inj: injured).

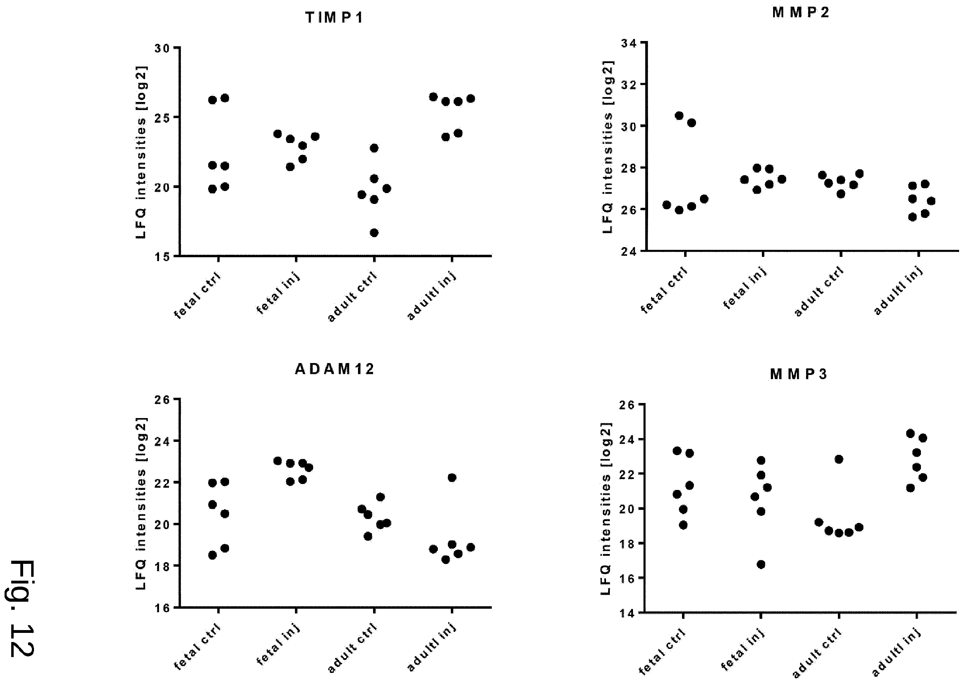

**Figure 12****:** From the abundance of inflammatory factors TIMP1, ADAM12, MMP2 and MMP3 in supernatants, several trends become apparent. (ctrl: control, inj: injured).

### Example 1 - Fetal articular cartilage regeneration versus adult fibrocartilaginous repair

This study aimed to 1) establish a standardized cartilage lesion model allowing comparison of cartilage healing in adult and fetal sheep (ovis aries); 2) establish the feasibility, repeatability and relevance of proteomic analysis of minute fetal and adult cartilage samples; and 3) compare fetal and adult protein regulation in response to cartilage injury.

The proteomic analysis of the differential response of fetal and adult cartilage to injury will have a major impact on our understanding of cartilage biology and of the molecular mechanisms underlying OA and cartilage regeneration, could help identify and target factors that are crucial to promote a regenerative response and allows the development of disease-modifying treatment strategies to induce cartilage regeneration in adult mammals. A major challenge to the proteomic characterization of the complex protein mixture in cartilage extract is the wide dynamic range of protein abundance, making the detection of low-abundant proteins very difficult (Stenberg et al., 2013; Wilson and Bateman, 2008). However, while technically demanding, studying the functional proteome gives a more comprehensive picture of disease aetiopathogenesis than gene expression analysis alone, as it can capture post-transcriptional regulation of protein expression levels as well as post-translational modifications (Ritter et al., 2013b).

### MATERIALS AND METHODS

### Animal Model

Standardized cartilage lesions in musculoskeletally mature (2-5 years, body weight 95±12kg), female, healthy, non-gravid Merino-cross ewes (ovis aries) without orthopaedic disease and fetal lambs (gestational day 80, term = 150 days) were created with approval of the national (Federal Ministry of Science, BMWFW) and institutional animal welfare committee. For the fetal subjects, only twin pregnancies were included to provide a twin lamb as uninjured control on a background of low genetic variation to allow differentiation between protein secretion of regular fetal development and fetal response to cartilage injury. Fetal hind limbs were exteriorized from the uterus via a standard ventral-midline laparotomy followed by an uterotomy over a randomly chosen uterine horn.

For the purpose of lesion induction, a minimally invasive medial parapatellar arthrotomy (Orth and Madry, 2013) was performed in both knees in adult and fetal sheep. A bilateral full-thickness cartilage lesion with a diameter of 7mm (adult sheep) resp. 1mm (fetal lamb) was created in the medial femoral condyle region (figure 1) using a custom- made precision surgical instrument (trocar with sleeve) for adult sheep and for fetal lambs a Versi-handle (Ellis Instruments, Madison, NJ, USA) with adjustable depth control, which was set at a lesion depth of 1mm.

To ensure that differences in the healing response between fetal and adult articular cartilage were not caused by differences in blood supply, which in fetal sheep is starting at an articular cartilage depth of 400 µm (Namba et al., 1998), we created 3 microfractures through the subchondral bone plate in adult cartilage defects to gain access to the bone marrow vasculature (Dorotka et al., 2005a). The arthrotomy was closed in 1 dermal layer using 6-0 monofilament nylon (Monosof, Covidien, Minneapolis, USA) in fetal lambs and in 3 layers using 2-0 monofilament absorbable polyester (Biosyn, Covidien) for the joint capsule and subcutaneous tissue and 2-0 monofilament nylon for the skin in adult sheep. Adult animals were allowed full weight-bearing immediately after surgery. All adult sheep were treated with antibiotics peri-operatively and received pain management. Pain management was provided with morphine to avoid anti-inflammatory drugs, which would influence the result of the study.

### Pilot Study

In a pilot study, as a proof of principle of fetal regeneration versus adult fibrocartilaginous repair, 3 adult and 3 fetal injured sheep were euthanized at 5 months (adult) respectively 28 days (fetal) postoperatively for macroscopic and histologic evaluation of the defect repair. At the time of euthanasia, digital photographs were taken and the articular cartilage surface and the cartilage defect healing response was macroscopically evaluated using the Osteoarthritis Research Society International (OARSI) recommendations for macroscopic scoring of cartilage damage in sheep, taking into account surface roughening, fibrillation, fissures as well as presence and size of osteophytes and erosions (Little et al., 2010). For fetal sheep the OARSI macroscopic score was size-adjusted by multiplying the adult lesion size cut-off values with 3.4/36.4, the ratio of the reported tibia length of fetal versus adult sheep (Mufti et al., 2000; Salami et al., 2011).

### Tissue Harvest

At day 3 after injury, samples were collected from 3 biological replicates per comparison group (adult injured, fetal injured, fetal uninjured twin control). In adult sheep, samples were also harvested from uninjured controls (n=3).

After macroscopic scoring, the medial femoral condyles were surgically excised and left and right knees were randomly assigned to mass spectrometry and histology. For mass spectrometry the (cartilage)-tissue remnants contained in the defect area and the cartilage rim surrounding the lesion (3mm width: adults, 1mm width in fetal sheep) were excised.

### Histology and Immunohistochemistry

For histological analysis the femoral condyles were fixed in 4% buffered formalin. Condyles from adult sheep were decalcified in 8% neutral EDTA. After embedding in paraffin, 4 µm-thick sections were cut and mounted on APES-glutaraldehyde-coated slides (3-aminopropyltriethoxysilane; Sigma-Aldrich, Vienna, Austria). Consecutive sections were stained with Haematoxylin and Eosin (H&E), and Safranin O.

For immunohistochemistry, sections were deparaffinised, rehydrated and endogenous peroxidase was blocked with 0.6% hydrogen peroxide in methanol (15 min at room temperature). Nonspecific binding of antibodies was prevented by incubation with 1.5% normal goat serum (Dako Cytomation, Glostrup, Denmark) in phosphate-buffered saline (PBS; 30 min at room temperature). Primary antibodies (anti-collagen type 2, anti-Ki67, anti-MMP9, and anti-MMP13; table 2) were incubated overnight at 4°C. An appropriate BrightVision Peroxidase system (Immunologic, Duiven, The Netherlands) was used and peroxidase activities were localized with diaminobenzidine (DAB; Sigma-Aldrich). Cell nuclei were counterstained with Mayer's haematoxylin.

Tissue from adult sheep mammary glands and human placenta served as positive controls. For negative controls, the primary antibody was omitted.

### Mass Spectrometry

The cartilage rim and the tissue remnants obtained from the lesion site were cultivated in serum-free RPMI medium (Gibco, Life Technologies, Austria) supplemented with 100U/ml penicillin and 100µg/ml streptomycin (ATCC, LGC Standards GmbH, Germany) for 6h under standard cell culture conditions (37°C and 5% CO2). Afterwards, medium was sterile-filtered through a 0.2 µm filter and precipitated overnight with ice-cold ethanol at -20° C. After precipitation, proteins were dissolved in sample buffer (7.5 M urea, 1.5 M thiourea, 4% CHAPS, 0.05% SDS, 100 mM dithiothreitol (DDT)) and protein concentrations were determined using Bradford assay (Bio-Rad-Laboratories, Munich, Germany).

Twenty microgram of each protein sample was used for a filter-aided digestion as described previously (Aukland, 1991; Aukland et al., 1997a; Aukland et al., 1997b; Bileck et al., 2014; Wisniewski et al., 2009). Briefly, 3 kD molecular weight cut-off filters (Pall Austria Filter GmbH) were conditioned with MS grade water (Millipore GmbH). Protein samples were concentrated on the pre-washed filter by centrifugation at 15000 g for 15 min. After reduction with DTT (5 mg/ml dissolved in 8 M guanidinium hydrochloride in 50 mM ammonium bicarbonate buffer (ABC buffer), pH 8) and alkylation with iodoacetamide (10 mg/ml in 8 M guanidinium hydrochloride in 50 mM ABC buffer), samples were washed and 1 µg trypsin was added prior to incubation at 37°C for 18 h. After enzymatic digestion, peptide samples were cleaned with C-18 spin columns (Pierce, Thermo Scientific, Germany), dried and stored at -20°C until analysis.

For mass spectrometric analyses, dried samples were reconstituted in 5 µl 30% formic acid (FA) containing 10 fmol of four synthetic standard peptides each and diluted with 40 µl mobile phase A (H2O:ACN:FA = 98:2:0.1). Ten microliter of the peptide solution were loaded onto a 2 cm x 75 µm C18 Pepmap100 pre-column (Thermo Fisher Scientific, Austria) at a flow rate of 10 µl/min using mobile phase A. Afterwards, peptides were eluted from the pre-column to a 50 cm x 75 µm Pepmap100 analytical column (Thermo Fisher Scientific, Austria) at a flow rate of 300 nl/min and separation was achieved using a gradient of 8% to 40% mobile phase B (ACN:H2O:FA = 80:20:0.1) over 95 min. For mass spectrometric analyses, MS scans were performed in the range of m/z 400-1400 at a resolution of 70000 (at m/z = 200). MS/MS scans of the 8 most abundant ions were achieved through HCD fragmentation at 30% normalized collision energy and analysed in the orbitrap at a resolution of 17500 (at m/z = 200). All samples were analysed in duplicates.

### Data analysis and statistics of mass spectrometry experiments

Protein identification as well as label-free quantitative (LFQ) data analysis was performed using the open source software MaxQuant 1.3.0.5 including the Andromeda search engine (Cox and Mann, 2008). Protein identification was achieved searching against ovis aries in the Uniprot Database (version 09/2014 with 26 864 entries) allowing a mass tolerance of 5 ppm for MS spectra and 20 ppm for MS/MS spectra as well as a maximum of 2 missed cleavages. In addition, carbamidomethylation on cysteins was included as fixed modification whereas methionine oxidation as well as N-terminal protein acetylation were included as variable modifications. Furthermore, search criteria included a minimum of two peptide identifications per protein, at least one of them unique, and the calculation based on q-values performed for both, peptide identification as well as protein identification, less than 0.01. Prior to statistical analyses, proteins were filtered for reversed sequences, contaminants and we required a minimum of three independent identifications per protein.

Missing values were replaced by a global ε, set to the minimum intensity observed in the entire data set divided by 4. This sensitivity based pseudo-count reflects the prior belief of non-observed protein expression, maintaining a lower bound of a 4-fold change for differences to proteins not observed in one sample group, thus maintaining sensitivity, while improving specificity by mitigating the effects of random non-observations. The Spearman rank correlations between samples shown in figure 6 are not affected by this transform. For the visualization of the sample correlation structure in that figure, ellipses were plotted as (x, y) = (cos(θ+d/2), cos(θ-d/2)), where θ in [0,2π) and cos(d)=ρ, with ρ the Spearman rank correlation coefficient (Murdoch and Chow, 1996).

Protein expression profile analysis was performed in the statistical environment R (www.r-project.org). Differential expression contrasts were computed with Bioconductor libraries (www.bioconductor.org). Data were normalized by a mean log-shift, standardizing mean expression levels per sample. Linear models were fit separately for each protein, computing second-level contrasts for a direct test of differences between fetal and adult responses to injury. Conservative Benjamini-Yekutieli correction was used to adjust for multiple testing to give strong control of the false discovery rate (FDR). We call significant features for q-values < 0.05. Linear models were adjusted for the nested correlation structure of technical and biological replicates (cf. figure 6). Significance was assessed by regularized t-tests. For these, group variances are shrunk by an Empirical Bayes procedure to mitigate the high uncertainty of variance estimates for the available sample sizes (Sun et al., 2009). The employed algorithms are implemented in the package limma (Smyth, 2005), which is available from Bioconductor.

### RESULTS

### The ovine model supports complex surgical manipulations required for the investigation of cartilage regeneration

Ewes and fetal sheep tolerated the laparotomy, uterotomy and fetal manipulation well. No postoperative complications or abortions were encountered. Fetal sheep at 80 days of gestation (gd) had age-appropriate crown-anus lengths within the reported range of 10.1 ±1.3 cm (Mufti et al., 2000). The landmarks for standardized induction of medial femoral condylar cartilage lesions (figure 1) were easily identified and allowed placement of the lesion in the centre of the condyle.

### Long-term evaluation confirmed fetal regenerative versus adult scarring cartilage repair

In the pilot study designed as a proof of principle of fetal regeneration at 28 days postoperatively versus adult scarring repair at 5 months post injury, the defect was macroscopically not detectable in fetal sheep resulting in an OARSI (Osteoarthritis Research Society International) macroscopic score (Little et al., 2010) of 0 for cartilage, osteophytes and synovium, while in adult sheep the defect was clearly evident and only partially filled with fibrocartilaginous tissue resulting in an OARSI macroscopic score of 5/16 for cartilage (surface roughening plus defect), 0/5 for osteophytes and 2/5 for synovium.

Histologically (figure 2), no defect repair and only minimal fibrocartilaginous regeneration adjacent to microfractures without integration with the surrounding cartilage was achieved in adult sheep 5 months postoperatively, whereas in fetal sheep, 28 days after surgery, the defect was filled with differentiating hyaline cartilage in about 80- 90% of the repair tissue and 10-20% with progressing hyalinisation and full integration with the surrounding cartilage.

### Injury- and repair-associated macroscopic and histologic changes in adult and fetal sheep 3 days post injury

Upon harvest at 3 days postoperatively, the OARSI macroscopic score was 4/16 for cartilage due to the 7mm (adults) resp. 1mm (fetal) size defect in the medial femoral condyle and 0 for osteophytes (due to the short time since surgery, no OARSI score was assigned to the macroscopic appearance of the synovium). Within the first 3 days after injury, no histologically visible cartilage repair could be detected. Therefore, none of the established repair scoring systems could be applied. Thus, the description of the structural conditions was based on the evaluation criteria of the ICRS assessment including the cartilage surface and matrix, cell distribution, cell viability, and subchondral bone but without scores (Mainil-Varlet et al., 2003).

Adult control condyles showed healthy articular cartilage with a smooth surface, physiologic matrix composition, and typical distribution of chondrocytes (figure 3A, B). Col2 staining was homogeneous and distinct throughout the whole articular cartilage (figure 3C).

Creation of the cartilage lesion in adults resulted in matrix depletion at the site of injury as well as in the superficial zone (figure 3D, E). Next to the cartilage lesion an acellular area of about 100 µm thickness was found with either empty lacunae or homogenous matrix lacking apparent lacunae. No cell clustering was observed. The microfractures penetrating the subchondral bone plate were visible (figure 3D, E). One sample showed a focal accumulation of granulocytes in the bone marrow cavity below the cartilage lesion. In the immediate vicinity (∼ 10µm) of the cartilage lesion, Col2 staining intensity was decreased (figure 3 F).

Similar to the adults, fetal uninjured control samples showed a smooth cartilage surface, homogenous matrix composition, and distinct Col2 staining throughout the whole condyles (figure 3G-I).

Although matrix depletion was also detected around the cartilage lesions in the fetal samples it was less marked compared to the adults (figure 3J, K). An almost acellular area of 50 µm surrounded the cartilage lesion. The lesion surface was partly covered with fibrous tissue originating either from cartilage canals or connective tissue flanking the articular surface. The pattern of the Col2 staining around the fetal cartilage lesions was similar to the adults with a 10 µm thick zone of decreased staining intensity (figure 3 L).

In adult control animals, no proliferating cells (demonstrated by expression of Ki67) were found in the articular cartilage or the subchondral bone (figure 4 A). Few Ki67-positive cells were detected in the bone marrow cavities. Chondrocytes in all cartilage zones expressed MMP9 and MMP13 with a stronger staining intensity for MMP9 (figure 4B, C), however no MMP-positive osteocytes were observed.

In the injured adult cartilage samples also no Ki67 positive cells were observed (figure 4 D). However, in one sample, an accumulation of Ki67-positive cells was found in a microfracture gap, which was filled with bone marrow. Both, MMP9 and MMP13-expression was reduced within and adjacent to the cartilage lesions (figure 4E, F) as compared to the intact cartilage of the respective sample.

In fetal healthy cartilage, evenly distributed Ki67-positive cells (figure 4 G) and MMP-expressing cells (figure 4H, I) were detected throughout the whole cartilage. The staining pattern of MMP9 appeared identical to MMP13.

Although, in the fetal injured cartilage an almost cell free zone of 50 µm was found to surround the lesions, single Ki67-expressing cells as well as MMP-positive cells could still be detected in this area (figure 4J-L). More MMP-expressing cells were located adjacent to the cell free zone as well as in the cartilage canals of the injured condyle.

### The ovine model supports comprehensive molecular profiling by high resolution mass spectrometry

Secretome analysis of control and injured (3 days postoperative) cartilage tissue samples derived from adult and fetal sheep, respectively, using high-resolution mass spectrometry (MS) enabled the identification of a total number of 2106 distinct proteins. Thereof, 445 proteins were found significantly regulated (q-value < 0.05) in response to cartilage injury in adult animals, in contrast to 74 proteins in fetal animals (figure 5). Comparing protein baseline expression, 1288 proteins were found significantly differentially regulated between fetal and adult control animals. The injury response of fetal and adult sheep was significantly differently regulated in 356 proteins. A comparison of protein regulation in adult and fetal animals (figure 5) revealed differences concerning the following groups of proteins: (i) proteins associated with immune response and inflammation, (ii) proteins specific for cartilage tissue and cartilage development and (iii) proteins involved in cell growth and proliferation (table 1). Multiple well-known actors in inflammatory processes, such as S100A8, S100A9, S100A12 and Ccdc88A were found significantly up-regulated following injury in adult (q< 0.001) but not in fetal animals (table 1). In contrast, several proteins with anti-inflammatory and growth-supporting effects, such as protein phosphatase, Mg2+/Mn2+ dependent 1A (Ppm1A) and cell division cycle 42 (Cdc42) showed a significant increase in response to injury in fetal sheep (q=0.005 and 0.006) compared to adults (table 1). Cartilage-specific proteins, such as aggrecan (Acan), cartilage oligomeric protein (Comp), chondroadherin (Chad) and proteoglycan-4 (Prg4) had a significantly higher baseline expression in adults (q<0.001) and showed little injury response in either age group with the exception of Prg4, which was significantly up-regulated in fetal injured sheep (q=0.01). Other proteins related to cell growth and proliferation, such as mitogen-activated protein kinase 3 (Mapk3/Erk1) and GA binding protein transcription factor alpha subunit (Gabpa), also displayed differences in abundance (q=0.02 and 0.04) as well as regulation between adult and fetal sheep (q=0.003 and 0.0001).

Our results demonstrate the biological relevance and reproducibility of our new ovine cartilage defect model and MS analysis (figure 6). Technical measurement reproducibility was excellent, with variation clearly lower than variation between biological replicates, indicating a high sensitivity of the proteomics profiling workflow (figure 6). The robustness of our new cartilage defect model is reflected in the variance across biological replicates being small in relation to the examined biological effects, whether injury versus control, or differences between adult and fetal samples (figure 6). For both adult and fetal samples, low variance across replicates indicates good reproducibility of our experimental setup, confirming that biologically meaningful signals can sensitively be obtained already from moderate sample size. Furthermore, it confirms good standardization of our articular cartilage defects between individuals of both the adult and fetal age group.

### DISCUSSION

The results illustrate the biological relevance, the technical feasibility and repeatability of the new ovine cartilage defect model (figure 1) and analytical approaches and confirm regeneration in fetal versus scarring repair in adult sheep (figure 2). Specific characteristics that make sheep particularly well-suited for OA, regenerative medicine and fetal regeneration research to obtain results of high clinical relevance are: 1) large size facilitating repeated sampling from individual animals and harvest of adequate sample sizes; 2) comparable bodyweight to humans; 3) similar mechanical exertion to humans (Bruns et al., 2000; Russo et al., 2015); 4) relatively long life expectancy (lifespan 8-12 years) allowing longitudinal analysis as well as evaluation of long-term efficacy and safety of treatments; 5) long gestational period (150 days) provides sufficient temporal resolution to translate findings obtained in sheep into human parameters (Jeanblanc et al., 2014); 6) extremely well characterized immune development analogous to humans; 7) bone marrow ontogeny and niche development closely paralleling humans.

Furthermore, for the sheep model, a quite acceptable annotation status and representative subsets of identified proteins are available on sources such as the NCBI (e.g. 30584 genes and 69889 proteins) allowing good applicability and translation of the results.

In this study we compared the adult and fetal response to cartilage injury 3 days after lesion induction as this time point is established to be within the time window of inflammation for both adult and fetal individuals, one of the injury responses hypothesized to crucially contribute to the difference between adult and fetal healing. For the fetal injury response, it is only known that cartilage regeneration occurs within 4 weeks, which is in stark contrast to the adult injury response with an inflammatory phase of 3-5 days, a proliferative phase of 3-6 weeks and a remodeling phase of up to one year duration resulting in a fibrocartilaginous scar. As the timeline of the fetal injury response is not yet established, choosing a later date would have made data interpretation and correlation of adult and fetal data much harder. Three days is within the peak period of the adult inflammatory response, allows for recruitment of monocytes/macrophages to the injury site and has been shown to be associated with signs of inflammation also in fetal injuries in other tissues.

The main factors identified within the secretome were extracellular matrix proteins, growth factors and inflammatory mediators such as cytokines and chemokines. Considering the key chondrocyte signalling pathways regulating processes of inflammation, cell proliferation, differentiation and matrix remodelling, which include the p38, Jnk and Erk Map kinases, the PI-3 kinase-Akt pathway, the Jak-Stat pathway, Rho GTPases and Wnt-β-catenin and Smad pathways (Beier and Loeser, 2010), the data provide an indication of differences in the inflammatory response to injury between adult and fetal cartilage and suggest the active production of anti-inflammatory and growth factors, such as Ppm1A and Cdc42 in the fetal environment.

Ppm1A is a bona fide phosphatase, which is involved in the regulation of many developmental processes such as skeletal and cardiovascular development. Through its role as phosphatase of many signalling molecules such as p38 kinase, Cdk2, phosphatidylinositol 3-kinase (PI3K), Axin and Smad, up-regulation of Ppm1A abolishes for example TGF-β-induced antiproliferative and transcriptional responses (Wang et al., 2014) as well as BMP signalling (Duan et al., 2006). Furthermore, Ppm1A by dephosphorylating IκB kinase-β and thus terminating TNFα-induced NF-κB activation, partakes in the regulation of inflammation, immune-response and apoptosis (Sun et al., 2009).

Cdc42 belongs to the family of Rho GTPases and controls a broad variety of signal transduction pathways regulating cell migration, polarization, adhesion proliferation, differentiation, and apoptosis in a variety of cell types (Sun et al., 2009). Cdc42 is required in successive steps of chondrogenesis by promoting mesenchymal condensation via the BMP2/Cdc42/Pak/p38/Smad signalling cascade and chondrogenic differentiation via the TGF-β/Cdc42/Pak/Akt/Sox9 signalling pathway (Wang et al., 2016). Another essential Cdc42 function relevant to the current study is its involvement in wound healing by attenuating MMP1 expression (Rohani et al., 2014) and regulating spatially distinct aspects of the cytoskeleton machinery, especially actin mobilization toward the wound (Benink and Bement, 2005) which, given the increase of actin-containing articular chondrocytes in response to cartilage injury, could also play a role in the healing of cartilage defects (Wang et al., 2000).

In contrast to the anti-inflammatory factors up-regulated in fetal sheep in response to injury, adult sheep displayed a significant increase of inflammatory mediators such as alarmins S100A8, S100A9, S100A12 and coiled-coil domain containing 88A (Ccdc88A). The alarmin S100 proteins are markers of destructive processes such as those occurring in OA (Liu-Bryan and Terkeltaub, 2015; Nefla et al., 2016; van den Bosch et al., 2015). Accordingly, in OA articular S100A8 and S100A9 protein secretion is increased, recruiting immune cells to inflamed synovia, initiating the adaptive immune response, inducing canonical Wnt signalling and promoting cartilage matrix catabolism, osteophyte formation, angiogenesis and hypertrophic differentiation (Liu-Bryan and Terkeltaub, 2015; Nefla et al., 2016; van den Bosch et al., 2015). S100A8/A9 up-regulate markers characteristic for ECM degradation (MMPs 1, 3, 9, and 13, interleukin-6 (IL-6), IL-8) and down-regulate growth promotion markers (aggrecan and Col2) and thus have a destructive effect on chondrocytes, causing proteoglycan depletion and cartilage breakdown (Schelbergen et al., 2012). Also S100A12 is up-regulated in OA cartilage and has been shown to increase the production of MMP-13 and Vegf in OA chondrocytes via p38 Mapk and NF-κB pathways (Nakashima et al., 2012). Another relevant protein, which was significantly down-regulated upon injury in fetal sheep but significantly up-regulated in injured adult sheep is Ccdc88A. Ccdc88A is a multimodular signal transducer, which modulates growth factor signalling during diverse biological and disease processes including cell migration, chemotaxis, development, self-renewal, apoptosis and autophagy by integrating signals downstream of a variety of growth factors, such as Efg, Igf, Vegf, Insulin, Stat3, Pdgfr and trimeric G protein Gi (Dunkel et al., 2012; Ghosh et al., 2008). In addition, Ccdc88A, which is expressed at high level in immune cells of the lymphoid lineage, plays an important role in T cell maturation, activation and cytokine production during pathological inflammation and its inhibition could help treat inflammatory conditions as shown in in-vitro and mouse studies (Kennedy et al., 2014). Furthermore Ccdc88A, via activation of Gαi, simultaneously enhances the profibrotic (Pi3k-Akt-FoxO1 and TGF-β-Smad) and inhibits the antifibrotic (cAMP-PKA-pCREB) pathways, shifting the fibrogenic signalling network toward a profibrotic programme (Lopez-Sanchez et al., 2014). Interestingly, in the liver, sustained up-regulation of Ccdc88A occurs only in all forms of chronic fibrogenic injuries but not in acute injuries that heal without fibrosis, indicating that increased expression of Ccdc88A during acute injuries may enhance progression to chronicity and fibrosis (Lopez-Sanchez et al., 2014). Ccdc88A also regulates the Pi3 kinase-Akt pathway, which exhibits pleiotropic functions in chondrogenesis, cartilage homeostasis and inflammation. It may further induce an increase in MMP production by chondrocytes leading to subsequent cartilage degeneration, via its multiple downstream target proteins (Chen et al., 2013; Fujita et al., 2004; Greene and Loeser, 2015; Kita et al., 2008; Litherland et al., 2008; Starkman et al., 2005; Xu et al., 2015).

Remarkably, in this study, the cartilage matrix proteins Prg4, Acan, Comp and Chad had a significantly higher baseline expression in adult sheep and showed little injury response in either age group with the exception of Prg4, which was significantly up-regulated in fetal injured sheep. Prg4, in response to injury increased 3.2 fold (q=0.01) in fetal sheep, which is a 4.6 fold higher increase compared to adults (q=0. 002), indicating a stronger and more rapid cartilage matrix production. Since Prg4 expressing cells constitute a cartilage progenitor cell population, the higher baseline expression in adults is particularly surprising but can be explained by its restriction to the most superficial cell layer in fetal joints compared to a distribution throughout the entire cartilage depth in older individuals (Kozhemyakina et al., 2015).

In contrast to the cartilage matrix glycoproteins, many growth factors, such as Gabpa and Mapk3 showed differential regulation following injury between adult and fetal sheep. Gabpa, a member of the ets protein family, which is ubiquitously expressed and plays an essential role in cellular functions such as cell cycle regulation, cellular growth, apoptosis, and differentiation (Rosmarin, 2004) showed a further significant up-regulation in fetal injury and no response to adult injury (q=0.0001). Gabpa activates the transcriptional co-activator Yes-associated protein (Yap), which is essential for cellular and tissue defences against oxidative stress, cell survival and proliferation and can induce the expression of growth-promoting genes important for tissue regeneration after injury (Wen Chun Juan, 2016). The cellular importance of Gabpa is further highlighted by the observation that in Gabpa conditional knockout embryonic stem cells (ESCs), disruption of Gabpa drastically repressed ESC proliferation and cells started to die within 2 days (Ueda et al., 2017).

The growth-regulator Mapk3 had a higher baseline expression in adult sheep (logFC=7.8, q=0>02) but significantly decreased (13.7 logFC, q<0.0001) after injury, while fetal Mapk3 remained essentially unchanged. Mapk3 acts as an essential component of the MAP kinase signal transduction pathway and as such contributes to cell growth, adhesion, survival and differentiation through the regulation of transcription, translation and cytoskeletal rearrangements. Mapk3 also fulfils an essential role in the control of chondrogenesis and osteogenesis of MSCs under TGF-β or mechanical induction and positively regulates chondrogenesis of MSCs (Bobick et al., 2010) .

The different inflammatory response as demonstrated herein in the fetus may be a major contributor to fetal scarless cartilage healing. This is especially surprising as fetal sheep have a normally functioning immune system by 75 gd (Almeida-Porada et al., 2004; Emmert et al., 2013). Leukocytes have been shown to be present and increase rapidly at the end of the first trimester (Mackay et al., 1986; Maddox et al., 1987d). Fetal sheep are able to form large amounts of specific antibodies in response to antigen stimuli by 70 gd (Silverstein et al., 1963) and reject orthotopic skin grafts and stem cell xenotransplants administered after 75-77 gd with the same competence and rapidity as adult (Silverstein et al., 1964). Furthermore, fetal sheep have an inflammatory response to injury before 80 gd (Kumta et al., 1994; Moss et al., 2008; Nitsos et al., 2016). The first evidence of inflammation, the presence of TNF and IL-1 has even been shown as early as 30 - 40 gd (Dziegielewska et al., 2000).

In conclusion, the results demonstrate the power of the new ovine fetal cartilage regeneration model and of the analytical approach. Both positive and negative regulators of inflammatory events were found to be differentially regulated, which holds promise for therapeutic interventions based on cell culture supernatants, in particular as the presence of a negative regulator is more easily mimicked than the absence of a positive regulator.

### TABLES

**Table 1: Selected relevant proteins, logFC represents the fold change in a logarithmic scale to the basis 2 based on label-free quantification (LFQ) intensities.**

| **Name** | **Fetal ctrl vs Adult ctrl** | | **Fetal D3 inj. vs ctrl** | | **Adult D3 inj. vs ctrl** | | **Fetal vs adult inj. response** | |
|---|---|---|---|---|---|---|---|---|
| | **logFC** | **q** | **logFC** | **q** | **logFC** | **q** | **logFC** | **q** |
| Acan | -10.74 | 5.54 | 1.77 | 1.00 | -1.36 | 9.15 | 3.13 | 1.01 |
| | | E-11 | | E+00 | | E-01 | | E-01 |
| Ccdc88A | 4.32 | 5.29 | -10.45 | 5.62 | 10.05 | 8.04 | -20.50 | 1.90 |
| | | E-01 | | E-03 | | E-04 | | E-05 |
| Ccdc42 | -3.58 | 5.97 | 8.68 | 4.27 | -3.45 | 9.39 | 12.13 | 5.47 |
| | | E-01 | | E-02 | | E-01 | | E-03 |
| Chad | -10.94 | 2.14 | 1.48 | 1.00 | -1.22 | 1.00 | 2.70 | 6.95 |
| | | E-09 | | E+00 | | E+00 | | E-01 |
| Col2a1 | -2.07 | 3.69 | 1.14 | 1.00 | -1.01 | 1.00 | 2.15 | 1.00 |
| | | E-01 | | E+00 | | E+00 | | E+00 |
| Comp | -9.64 | 1.13 | 1.67 | 1.00 | -0.05 | 1.00 | 1.72 | 1.00 |
| | | E-10 | | E+00 | | E+00 | | E+00 |
| Gabpa | -3.01 | 3.50 | 8.23 | 1.52 | -0.29 | 1.00 | 8.52 | 1.09 |
| | | E-02 | | E-05 | | E+00 | | E-04 |
| Mapk3 | -7.81 | 1.50 | 1.11 | 1.00 | -13.73 | 1.23 | 14.84 | 2.55 |
| | | E-02 | | E+00 | | E-05 | | E-03 |
| Ppm1A | -1.62 | 1.00 | 8.91 | 1.36 | -0.21 | 1.00 | 9.12 | 4.69 |
| | | E+00 | | E-03 | | E+00 | | E-03 |
| Prg4 | -11.56 | 3.94 | 3.18 | 1.27 | -1.43 | 5.29 | 4.61 | 1.63 |
| | | E-12 | | E-02 | | E-01 | | E-03 |
| S100A12 | -2.71 | 1.86 | 8.39 | 4.99 | 13.54 | 7.37 | -5.15 | 7.15 |
| | | E-01 | | E-05 | | E-10 | | E-02 |
| S100A8 | -2.78 | 3.48 | 7.49 | 1.29 | 15.80 | 7.15 | -8.32 | 3.53 |
| | | E-01 | | E-03 | | E-10 | | E-03 |
| S100A9 | 0.08 | 1.00 | 6.34 | 4.25 | 15.45 | 9.32 | -9.11 | 4.15 |
| | | E+00 | | E-01 | | E-08 | | E-02 |

**Table 2: Sources, pre-treatments and dilutions of the antibodies used for histology.**

| **Antibody** | **Clone** | **Concentration (v/v)** | **Pre-treatment** | **Source** |
|---|---|---|---|---|
| Col2 | 2B1.5 | 1/100 | 0.04% hyaluronidase (Sigma Aldrich) in PBS^{#}, 4 h at 37°C; followed by 1% protease (Sigma Aldrich) in PBS, 30 min at 37°C | Thermo Fisher Scientific, Waltham, MA |
| Ki67 | SP6 | 1/400 | 0.01M citrate buffer pH 6.0, 2 h at 85°C | Thermo Fisher Scientific |
| Mmp9 | poly | 1/100 | 0.01M citrate buffer pH 6.0, 30 min at 90°C - 95°C | Abnova, Heidelberg, Germany |
| Mmp13 | poly | 1/50 | 0.01M citrate buffer pH 6.0, 30 min at 90°C - 95°C | Thermo Fisher Scientific |

### Example 2 - Cell culture supernatant of tendon cells obtained from adult and fetal sheep

In adult (2-4 years of age) and fetal (day 80 of gestation) sheep, surgical lesions were induced at a tendon (see Fig. 7). Tendon samples were collected before lesion induction and on day 3 after lesion induction (leading to four sample groups: adult control, adult injured, fetal control, fetal injured). Cell culture, supernatant collection and fractionation, and mass-spectrometric secretome analysis was performed essentially as in Example 1. Many proteins in the secretome were diffentially secreted into the supernatant between the sample groups. A selection of relevant examples is shown in Figs. 8 to 12.

The results demonstrated large differences between supernatants of cell cultures obtained from fetal and adult tendon, implying therapeutic relevance of the fetal supernatants due to the much higher regeneration potential of injured fetal tendon.

Accordingly, the present invention discloses the following preferred embodiments:
1. A method for obtaining a fraction of a fetal cell culture supernatant, comprising the steps of
   - obtaining a cell-containing sample of tissue from a non-human mammalian fetus,
   - culturing the sample in a liquid cell culture medium, thereby obtaining a cell culture with a liquid supernatant, and
   - isolating a fraction from the supernatant.
2. The method of embodiment 1, wherein the fetus is within the first two trimesters of gestation, preferably within the first half of gestation.
3. The method of any one of embodiments 1 to 2, wherein the sample comprises chondrocytes, chondroblasts, chondroprogenitor cells, tenocytes, tenoblasts, tendon progenitor cells, fibrocytes, fibroblasts, fibrochondrocytes, fibrochondroblasts, synoviocytes, synovioblasts, osteocytes, osteoblasts, osteoclasts, hepatocytes, monocyte, macrophage, mesenchymal stem cells, mesenchymal progenitor cells and/or interzone cells.
4. The method of any one of embodiments 1 to 3, wherein the tissue is selected from cartilage, tendon, ligament, bone, bone marrow and blood, in particular cord-blood.
5. The method of any one of embodiments 1 to 4, wherein the tissue is articular cartilage.
6. The method of any one of embodiments 1 to 5, wherein the culturing step comprises injuring, preferably chemically or mechanically injuring, the cells.
7. The method of any one of embodiments 1 to 6, wherein the fraction comprises proteins, lipids, metabolites, extracellular vesicles and/or RNA, in particular miRNA.
8. The method of any one of embodiments 1 to 7, wherein the liquid cell culture medium is a serum-free cell culture medium, a protein-free cell culture medium or a chemically defined cell culture medium.
9. The method of any one of embodiments 1 to 8, wherein the isolating step comprises a sterile filtration.
10. The method of any one of embodiments 1 to 9, wherein the isolating step comprises adding a protein-precipitating agent.
11. The method of any one of embodiments 1 to 10, wherein the isolating step comprises a centrifugation step.
12. The method of any one of embodiments 1 to 11, wherein the isolating step comprises a preservation step, preferably a freezing or drying step, especially lyophilisation.
13. The method according to any one of embodiments 1 to 12, wherein the cell culture medium comprises fetal calf serum (FCS) or other nutrient additives.
14. A fraction, obtainable by the method of any one of embodiments 1 to 13.
15. A cell supernatant fraction from non-human fetal cells, preferably wherein the cells are defined as in embodiment 3, wherein the fraction comprises proteins, lipids, metabolites, extracellular vesicles and/or RNA, in particular miRNA.
16. The fraction of embodiment 14 or 15, wherein the fraction is dry, preferably lyophilised.
17. A pharmaceutical composition, comprising the fraction of any one of embodiments 14 to 16.
18. The pharmaceutical composition of embodiment 17 for use in therapy.
19. The pharmaceutical composition of embodiment 18 for use in a prevention or treatment of osteoarthritis, arthritis, tendinitis, tendinopathy, cartilage injury, tendon injury, rheumatoid arthritis, discospondylitis, meniscus injury, desmitis, desmopathy, intervertebral disc injuries, degenerative disease of intervertebral discs, reperfusion injury, wounds or inflammatory disease.

### NON-PATENT REFERENCES

Al-Qattan, et al. (1993). Fetal Tendon Healing: Development of an Experimental Model. Plastic and Reconstructive Surgery 92, 1155.
Aukland (1991). Distribution volumes and macromolecular mobility in rat tail tendon interstitium. Am. J. Physiol. 260, H409-19.
Aukland, et al. (1997a). The problem of gaining access to interstitial fluid. An attempt to rationalize a wicked discussion on wicks. Lymphology 30, 111-115.
Aukland, et al. (1997b). Interstitial exclusion of macromolecules studied by graded centrifugation of rat tail tendon. Am. J. Physiol. 273, H2794-803.
Beier, et al. (2010). Biology and pathology of Rho GTPase, PI-3 kinase-Akt, and MAP kinase signaling pathways in chondrocytes. J. Cell. Biochem. 110, 573-580.
Benink, et al. (2005). Concentric zones of active RhoA and Cdc42 around single cell wounds. The Journal of Cell Biology 168, 429-439.
Bileck, et al. (2014). Comprehensive assessment of proteins regulated by dexamethasone reveals novel effects in primary human peripheral blood mononuclear cells. J. Proteome Res. 13, 5989-6000.
Bobick, et al. (2010). The ERK5 and ERK1/2 signaling pathways play opposing regulatory roles during chondrogenesis of adult human bone marrow-derived multipotent progenitor cells. J. Cell. Physiol. n/a-n/a.
Bruns, et al. (2000). Achilles tendon rupture: experimental results on spontaneous repair in a sheep-model. Knee surgery, sports traumatology, arthroscopy 8, 364-369.
Chen, et al. (2013). Vertical inhibition of the PI3K/Akt/mTOR pathway for the treatment of osteoarthritis. J. Cell. Biochem. 114, 245-249.
Cowin, et al. (1998b). Endogenous inflammatory response to dermal wound healing in the fetal and adult mouse. Developmental dynamics 212, 385-393.
Degen, et al. (2012). Embryonic wound healing: A primer for engineering novel therapies for tissue repair. Birth Defect Res C 96, 258-270.
Decker, et al. (2017). Cell origin, volume and arrangement are drivers of articular cartilage formation, morphogenesis and response to injury in mouse limbs. Developmental Biology 426, 56-68.
Dorotka, et al. (2005a). Marrow stimulation and chondrocyte transplantation using a collagen matrix for cartilage repair. Osteoarthritis and cartilage 13, 655-664.
Duan, et al. (2006). Protein serine/threonine phosphatase PPM1A dephosphorylates Smad1 in the bone morphogenetic protein signaling pathway. The Journal of biological chemistry 281, 36526-36532.
Dunkel, et al. (2012). STAT3 protein up-regulates Gα-interacting vesicle-associated protein (GIV)/Girdin expression, and GIV enhances STAT3 activation in a positive feedback loop during wound healing and tumor invasion/metastasis. Journal of Biological Chemistry 287, 41667-41683.
Dziegielewska, et al. (2000). Acute-phase cytokines IL-lbeta and TNF-alpha in brain development. Cell Tissue Res 299, 335-345.
Emmert, et al. (2013). Intramyocardial transplantation and tracking of human mesenchymal stem cells in a novel intra-uterine pre-immune fetal sheep myocardial infarction model: a proof of concept study. PLoS ONE 8, e57759.
Ferguson, et al. (2004). Scar-free healing: from embryonic mechanisms to adult therapeutic intervention. Philosophical Transactions of the Royal Society B: Biological Sciences 359, 839-850.
Fujita, et al. (2004). Runx2 induces osteoblast and chondrocyte differentiation and enhances their migration by coupling with PI3K-Akt signaling. The Journal of Cell Biology 166, 85-95.
Ghosh, et al. (2008). Activation of Galphai3 triggers cell migration via regulation of GIV. The Journal of Cell Biology 182, 381-393.
Greene, et al. (2015). Function of the chondrocyte PI-3 kinase-Akt signaling pathway is stimulus dependent. Osteoarthritis and cartilage 23, 949-956.
Jeanblanc, et al. (2014). Temporal definition of haematopoietic stem cell niches in a large animal model of in utero stem cell transplantation. British Journal of Haematology 166, 268-278.
Jenner, et al. (2014). Differential gene expression of the intermediate and outer interzone layers of developing articular cartilage in murine embryos. Stem Cells and Development 23, 1883-1898.
Jenner, et al. Laser capture microdissection of murine interzone cells: layer selection and prediction of RNA yield. J Stem Cell Res Ther 4, 1000183.
Johnson, et al. (2014). The epidemiology of osteoarthritis. Best Practice & Research Clinical Rheumatology 28, 5-15.
Kennedy, et al. (2014). CCDC88B is a novel regulator of maturation and effector functions of T cells during pathological inflammation. J. Exp. Med. 211, 2519-2535.
Kita, et al. (2008). PI3K/Akt signaling as a key regulatory pathway for chondrocyte terminal differentiation. Genes to Cells 13, 839-850.
Kozhemyakina, et al. (2015). Identification of a Prg4-expressing articular cartilage progenitor cell population in mice. Arthritis Rheumatol 67, 1261-1273.
Kumahashi, et al. (2004). Involvement of ATP, increase of intracellular calcium and the early expression of c-fos in the repair of rat fetal articular cartilage. Cell Tissue Res 317, 117-128.
Kumta, et al. (1994). Acute inflammation in foetal and adult sheep: the response to subcutaneous injection of turpentine and carrageenan. Br J Plast Surg 47, 360-368.
Litherland, et al. (2008). Synergistic collagenase expression and cartilage collagenolysis are phosphatidylinositol 3-kinase/Akt signaling-dependent. The Journal of biological chemistry 283, 14221-14229.
Little, et al. (2010). The OARSI histopathology initiative - recommendations for histological assessments of osteoarthritis in sheep and goats. Osteoarthritis and cartilage 18 Suppl 3, S80-92.
Liu-Bryan, et al. (2015). Emerging regulators of the inflammatory process in osteoarthritis. Nature Reviews Rheumatology 11, 35-44.
Longaker, et al. (1990). Studies in fetal wound healing VI. Second and early third trimester fetal wounds demonstrate rapid collagen deposition without scar formation. J. Pediatr. Surg. 25, 63-69.
Longaker, et al. (1990). Studies in fetal wound healing VI. Second and early third trimester fetal wounds demonstrate rapid collagen deposition without scar formation. J. Pediatr. Surg. 25, 63-69.
Lo, et al. (2012). Scarless fetal skin wound healing update. Birth Defect Res C 96, 237-247.
Lopez, et al. (1998). The global burden of disease, 1990-2020. Nat Med 4, 1241-1243.
Lopez-Sanchez, et al. (2014). GIV/Girdin is a central hub for profibrogenic signalling networks during liver fibrosis. Nat Comms 5, 4451.
Mackay, et al. (1986). Thymocyte subpopulations during early fetal development in sheep. Journal of immunology 136, 1592-1599.
Maddox, et al. (1987a). Ontogeny of ovine lymphocytes. I. An immunohistological study on the development of T lymphocytes in the sheep embryo and fetal thymus. Immunology 62, 97-105.
Maddox, et al. (1987b). Ontogeny of ovine lymphocytes. II. An immunohistological study on the development of T lymphocytes in the sheep fetal spleen. Immunology 62, 107-112.
Maddox, et al. (1987c). Ontogeny of ovine lymphocytes. III. An immunohistological study on the development of T lymphocytes in sheep fetal lymph nodes. Immunology 62, 113-118.
Maddox, et al. (1987d). Ontogeny of ovine lymphocytes. III. An immunohistological study on the development of T lymphocytes in sheep fetal lymph nodes. Immunology 62, 113-118.
Mainil-Varlet, et al. (2003). Histological assessment of cartilage repair: a report by the Histology Endpoint Committee of the International Cartilage Repair Society (ICRS). J Bone Joint Surg Am 85-A Suppl 2, 45-57.
Moss, et al. (2008). Experimental amniotic fluid infection in sheep: Effects of Ureaplasma parvum serovars 3 and 6 on preterm or term fetal sheep. American Journal of Obstetrics and Gynecology 198, 122.e1-122.e8.
Mufti, et al. (2000). Prenatal development of ovine fetus. Small Ruminant Research 38, 87-89.
Murdoch, et al. (1996). A Graphical Display of Large Correlation Matrices. The American Statistician 50, 178.
Nakashima, et al. (2012). Role of S100A12 in the pathogenesis of osteoarthritis. Biochemical and Biophysical Research Communications 422, 508-514.
Namba, et al. (1998). Spontaneous repair of superficial defects in articular cartilage in a fetal lamb model. J Bone Joint Surg Am 80, 4-10.
Nefla, et al. (2016). The danger from within: alarmins in arthritis. Nature Reviews Rheumatology 12, 669-683.
Nitsos, et al. (2016). Chronic exposure to intra-amniotic lipopolysaccharide affects the ovine fetal brain. Reproductive Sciences 13, 239-247.
Orth, et al. (2013). A low morbidity surgical approach to the sheep femoral trochlea. BMC Musculoskelet Disord 14, 1511-8.
Rohani, et al. (2014). Cdc42 inhibits ERK-mediated collagenase-1 (MMP-1) expression in collagen-activated human keratinocytes. J. Invest. Dermatol. 134, 1230-1237.
Ritter, et al. (2013b). Proteomic Analysis of Synovial Fluid From the Osteoarthritic Knee: Comparison With Transcriptome Analyses of Joint Tissues. Arthritis & Rheumatism 65, 981-992.
Rosmarin. (2004). GA-binding protein transcription factor: a review of GABP as an integrator of intracellular signaling and protein-protein interactions. Blood Cells, Molecules, and Diseases 32, 143-154.
Russo, et al. (2015). Cellular and molecular maturation in fetal and adult ovine calcaneal tendons. J Anatomy 226, 126-142.
Salami, et al. (2011). Comparative Osteometric Study of Long Bones in Yankasa Sheep and Red Sokoto Goats . International Journal of Morphology 29, 100-104.
Schelbergen, et al. (2012). Alarmins S100A8 and S100A9 elicit a catabolic effect in human osteoarthritic chondrocytes that is dependent on Toll-like receptor 4. Arthritis & Rheumatism 64, 1477-1487.
Silverstein, et al. (1964). Fetal response to antigenic stimulus. IV. Rejection of skin homografts by the fetal lamb. J. Exp. Med. 119, 955-964.
Silverstein, et al. (1963). Fetal response to antigenic stimulus. II. Antibody production by the fetal lamb. J. Exp. Med. 117, 799-812.
Smyth. (2005). limma: Linear Models for Microarray Data. In Bioinformatics and Computational Biology Solutions Using R and Bioconductor, pp. 397-420. New York: Springer, New York, NY.
Starkman, et al. (2005). IGF-I stimulation of proteoglycan synthesis by chondrocytes requires activation of the PI 3-kinase pathway but not ERK MAPK. Biochem. J. 389, 723-729.
Stenberg, et al. (2013). Quantitative proteomics reveals regulatory differences in the chondrocyte secretome from human medial and lateral femoral condyles in osteoarthritic patients. Proteome Science 11, 1-1.
Stone. (2000). Unravelling the secrets of foetal wound healing: an insight into fracture repair in the mouse foetus and perspectives for clinical application. Br J Plast Surg 53, 337-341.
Sun, et al. (2009). PPM1A and PPM1B act as IKKβ phosphatases to terminate TNFα-induced IKKβ-NP-κB activation. Cellular Signalling 21, 95-102.
Sun et al., Biomaterials 32 (2011), 5581-5589
Pap, et al. (2015). Cartilage damage in osteoarthritis and rheumatoid arthritis-two unequal siblings. Nature Reviews Rheumatology 11, 606-615
Ueda, et al. (2017). GA-Binding Protein Alpha Is Involved in the Survival of Mouse Embryonic Stem Cells. Stem Cells 306, 391.
van den Bosch, et al. (2015). Induction of Canonical Wnt Signaling by the Alarmins S100A8/A9 in Murine Knee Joints: Implications for Osteoarthritis. Arthritis & Rheumatology 68, 152-163.
Wagner, et al. (2001). Neonatal rat cartilage has the capacity for tissue regeneration. Wound Repair Regen 9, 531-536.
Walker, et al. (2000). Cellular responses of embryonic hyaline cartilage to experimental wounding in vitro. Journal of orthopaedic research 18, 25-34.
Wang, et al. (2016). Signaling Cascades Governing Cdc42-Mediated Chondrogenic Differentiation and Mensenchymal Condensation. Genetics 202, 1055-1069.
Wang, et al. (2000). Healing of defects in canine articular cartilage: distribution of nonvascular alpha-smooth muscle actin-containing cells. Wound repair and regeneration 8, 145-158.
Wen Chun Juan, et al. (2016). Targeting the Hippo Signaling Pathway for Tissue Regeneration and Cancer Therapy. Genes 7, 55.
Wilson, et al. (2008). Cartilage proteomics: Challenges, solutions and recent advances. Prot. Clin. Appl. 2, 251-263.
Wiśniewski, et al. (2009). Universal sample preparation method for proteome analysis. Nat Meth 6, 359-362.
Xu, et al. (2015). Osteopontin induces vascular endothelial growth factor expression in articular cartilage through PI3K/AKT and ERK1/2 signaling. Molecular Medicine Reports 12, 4708-4712.

## Claims

1. A method for obtaining a fraction of a fetal cell culture supernatant, comprising the steps of
- obtaining a cell-containing sample of tissue from a non-human mammalian fetus,
- culturing the sample in a liquid cell culture medium, thereby obtaining a cell culture with a liquid supernatant, and
- isolating a fraction from the supernatant.

2. The method of claim 1, wherein the fetus is within the first two trimesters of gestation, preferably within the first half of gestation.

3. The method of any one of claims 1 to 2, wherein the sample comprises chondrocytes, chondroblasts, chondroprogenitor cells, tenocytes, tenoblasts, tendon progenitor cells, fibrocytes, fibroblasts, fibrochondrocytes, fibrochondroblasts, synoviocytes, synovioblasts, osteocytes, osteoblasts, osteoclasts, hepatocytes, monocyte, macrophage, mesenchymal stem cells, mesenchymal progenitor cells and/or interzone cells.

4. The method of any one of claims 1 to 3, wherein the tissue is selected from cartilage, tendon, ligament, bone, bone marrow and blood, in particular cord-blood.

5. The method of any one of claims 1 to 4, wherein the tissue is articular cartilage.

6. The method of any one of claims 1 to 5, wherein the culturing step comprises injuring, preferably chemically or mechanically injuring, the cells.

7. The method of any one of claims 1 to 6, wherein the fraction comprises proteins, lipids, metabolites, extracellular vesicles and/or RNA, in particular miRNA.

8. The method of any one of claims 1 to 7, wherein the liquid cell culture medium is a serum-free cell culture medium, a protein-free cell culture medium or a chemically defined cell culture medium.

9. The method of any one of claims 1 to 8, wherein the isolating step comprises a preservation step, preferably a freezing or drying step, especially lyophilisation.

10. A fraction, obtainable by the method of any one of claims 1 to 9.

11. A cell supernatant fraction from non-human fetal cells, preferably wherein the cells are defined as in claim 3, wherein the fraction comprises proteins, lipids, metabolites, extracellular vesicles and/or RNA, in particular miRNA.

12. The fraction of claim 10 or 11, wherein the fraction is dry, preferably lyophilised.

13. A pharmaceutical composition, comprising the fraction of any one of claims 10 to 12.

14. The pharmaceutical composition of claim 13 for use in therapy.

15. The pharmaceutical composition of claim 14 for use in a prevention or treatment of osteoarthritis, arthritis, tendinitis, tendinopathy, cartilage injury, tendon injury, rheumatoid arthritis, discospondylitis, meniscus injury, desmitis, desmopathy, intervertebral disc injuries, degenerative disease of intervertebral discs, reperfusion injury, wounds or inflammatory disease.
